# EUROPÄISCHE PATENTSCHRIFT

(11) **EP 0 421 211 B1**
(45) Veröffentlichungstag und Bekanntmachung des Hinweises auf die Patenterteilung: **19.01.1994**
(21) Anmeldenummer: 90118243.6
(22) Anmeldetag: 22.09.1990
(51) Int. Cl.: C12M 3/00

(54) **Reaktor zur Durchführung von biologischen in vitro-Prozessen, Verfahren zur Durchführung eines biologischen in vitro-Prozesses und Verwendung des Reaktors und des Verfahrens**
Reactor for carrying out biological processes in-vitro, method for carrying out biological processes in-vitro and use of the reactor and method
Réacteur pour la mise en oeuvre de procédés biologiques in-vitro, méthode pour la mise en oeuvre de procédés biologiques et utilisation du réacteur et de la méthode

(30) Priorität: 29.09.1989 DE 3932633
(43) Veröffentlichungstag der Anmeldung: 10.04.1991
(73) Patentinhaber: Anawa München Aktiengesellschaft Biologische Laboratorien, D-82152 Planegg (DE)
(72) Erfinder: Müller-Lierheim, Wolfgang Dr., W-8000 München 71 (DE); Wittik, Bernd Dr., W-8300 Altdorf (DE)
(74) Vertreter: Nöth, Heinz, Dipl.-Phys.

(56) Entgegenhaltungen:
- EP-A- 0 205 790
- EP-A- 0 253 774
- WO-A-86/02944

## Beschreibung

Die Erfindung betrifft einen Reaktor und ein Verfahren zur Durchführung von biologischen in vitro-Prozessen, insbesondere Zellkultivierung, Stoffwechselvorgängen und Behandlung von fließfähigen Medien.

Zur Kultivierung von menschlichen und/oder tierischen Zellen ist aus der EP-A-205 790 ein Kultivierungsgefäß bzw. Fermenter bekannt, bei dem ein mit Wachstumsfaktoren überzogener partikelförmiger Füllstoff in Form eines Perlpolymerisats in mehreren Schichten übereinander angeordnet wird. Die Perlpolymerisatschichten werden vom Zellkulturmedium durchströmt, und man gewinnt ein Zellwachstum mit hoher Zelldichte.

Aus EP-A-253774 ist ein Fermenter mit einem Rührwerk bekannt, das an einer drehbaren axialen Welle mit in Abstand zueinander angeordneten Scheiben aufweist, auf denen Zellen immobilisiert sind.

Aufgabe der Erfindung ist es, einen Reaktor, ein Verfahren sowie eine Verwendung von Reaktor und Verfahren der eingangs genannten Art, zu schaffen, bei denen gewährleistet ist, daß die an die Füllstoffschichten angewachsenen Zellen haften bleiben, d. h. insbesondere scherkraftarme Bedingungen gewährleistet sind.

Diese Aufgabe wird beim Reaktor erfindungsgemäß durch die Merkmale des Anspruchs 1 und beim Verfahren durch die Merkmale des Anspruchs 6 gelöst.

Durch den erfindungsgemäßen Reaktor wird eine schichtförmige Anordnung des partikelförmigen Füllstoffs gewonnen, bei dem die Füllstoffschichten in scheibenförmigen Paketen in bestimmtem Abstand voneinander längs der Reaktionsgefäßachse parallel zueinander fixiert sind. Die Umhüllung, welche die jeweiligen Füllstoffschichten umgibt und diese zusammenhält, erlaubt eine Diffusion des fließfähigen Mediums, das ein Nährmedium oder ein zu behandelndes Medium wie z.B. zu reinigendes Blut sein kann, und gegebenenfalls auch die Diffusion von Zellen. Der partikelförmige Füllstoff kann in vertikal zur horizontal liegenden Gefäßachse angeordneten Schichten, die in horizontaler Richtung nebeneinander liegen, im Gefäßinnern des Reaktors angeordnet werden. Das Reaktionsgefäß ist mit dem Medium, in welchem der biologische Prozeß jeweils durchgeführt werden soll, zum Teil angefüllt, und durch Drehen der auf der Trägerwelle fixierten Füllstoffschichten gegenüber dem Medium bleibt gewährleistet, daß der Füllstoff in den Umhüllungen als jeweilige Schicht bzw. Paket erhalten bleibt. Auf Grund der eine Diffusion des Mediums und/oder der Zellen ermöglichenden Eigenschaft der Umhüllung werden scherkraftarme Bedingungen für den Reationsablauf gewonnen, die insbesondere für ein ungehindertes Zellwachstum und ein Anhaften der Zellen an den Füllstoffpartikeln vorteilhaft sind. Gleichzeitig ist eine Durchdringung der Zwischenräume zwischen Füllstoffpartikeln mit dem Medium gewährleistet. Die Zellen, die in den Zwischenräumen zwischen den Füllstoffpartikeln gewachsen sind, werden durch Diffusion ständig mit Nährmedium versorgt.

Es ist auch möglich, das Reaktionsgefäß mit vertikaler Gefäßachse anzuordnen, so daß dann die Füllstoffpartikelschichten sich parallel zueinander in horizontaler Richtung erstrecken und längs der vertikalen Gefäßachse übereinander mit bestimmtem Abstand voneinander, angeordnet sind.

Von Vorteil ist außerdem, daß das Befüllen der Umhüllungen mit dem partikelförmigen Füllstoff bei nichtsterilen Bedingungen erfolgen kann. Nach dem Einsetzen der an der Trägerwelle fixierten Füllstoffschichten in das Reaktionsgefäß kann nämlich das Innere des Reaktionsgefäßes mit den Füllstoffschichten autoklaviert werden und anschließend mit dem Zellkulturmedium beschickt werden.

Das Reaktionsgefäß kann nach Art einer Rollerflasche ausgebildet sein, wobei die Trägerwelle mit den daran befestigten Füllstoffschichten zusammen mit dem Reaktionsgefäß gegenüber dem Medium gedreht werden.

Es ist auch möglich, die Trägerwelle zusammen mit den daran fixierten Füllstoffschichten gegenüber dem Reaktionsgefäß und dem im Reaktionsgefäß befindlichen Medium zu drehen. Hierzu ist dann die Trägerwelle am Reaktionsgefäß drehbar gelagert.

Durch die Drehung der Füllstoffschichten gegenüber dem im Reaktionsgefäß befindlichen Medium wird ferner ein Rühreffekt erreicht, durch den eine homogene Verteilung der im Medium, insbesondere im Zellkulturmedium gelösten Stoffe wie in einem Rührkessel erreicht wird.

Bevorzugt werden als Umhüllungen für die jeweiligen Schichten der Füllstoffpartikel Haltesiebe, bevorzugt aus rostfreiem Stahl oder Teflon verwendet. Diese Haltesiebe besitzen eine Maschenweite, welche eine Diffusion des Zellkulturmediums bzw. des fließfähigen Mediums, welches behandelt werden soll, durch die Siebwand zu den Füllstoffpartikeln erlaubt.

Bevorzugt beträgt die Maschenweite der Haltesiebe 40 Mikron (µm) bis 100 Mikron (µm).

Als partikelförmiger Füllstoff kommt bevorzugt ein Perlpolymerisat zum Einsatz, dessen Perlgröße >40 Mikron (µm), bis zu 300 Mikron (µm) ist. Eine Durchschnittsdurchmessergröße von etwa 150 bis 200 Mikron (µm) wird bevorzugt. Die Schichtdicken der paketförmigen Schichten des Perlpolymerisats, das durch die Haltesiebe zusammengehalten wird, beträgt bevorzugt 1000 Mikron (µm) bis 2000 Mikron (µm). Ein verwendbares Polymerisat ist aus der EP-A-205 790 bekannt.

In bevorzugter Weise wird ein Wachstum von Säugetierzellen, insbesondere Humanzellen auf dem Perlpolymerisat bzw. Füllstoffpartikeln durchgeführt. Nach Entfernen des Kulturmediums kann dann die Behandlung eines fließfähigen Mediums, das an den mit den Zellen bewachsenen Füllstoffschichten im Reaktionsgefäß vorbeigeleitet wird, erfolgen.

Wenn Hepatozyten bzw. Leberzellen kultiviert worden sind, eignet sich das Reaktionsgefäß zur Blutreinigung. Das Reationsgefäß kann dann als künstliche Leber eingesetzt werden.

Anhand der beiliegenden Figuren wird an einem Ausführungsbeispiel die Erfindung noch näher erläutert. Es zeigt:
- Fig. 1: in schnittbildlicher Darstellung eines ersten Ausführungsbeispiels eines Reaktors;
- Fig. 2: eine Teilansicht einer von einem Haltesieb gehaltenen Füllstoffpartikelschicht von der Seite;
- Fig. 3: eine Ansicht des in der Fig. 2 dargestellten schichtförmigen Schichtstoffpakets in Blickrichtung eines Pfeiles A in Fig. 2.
- Fig. 4: ein zweites Ausführungsbeispiel und
- Fig. 5: ein drittes Ausführungsbeispiel der Erfindung.

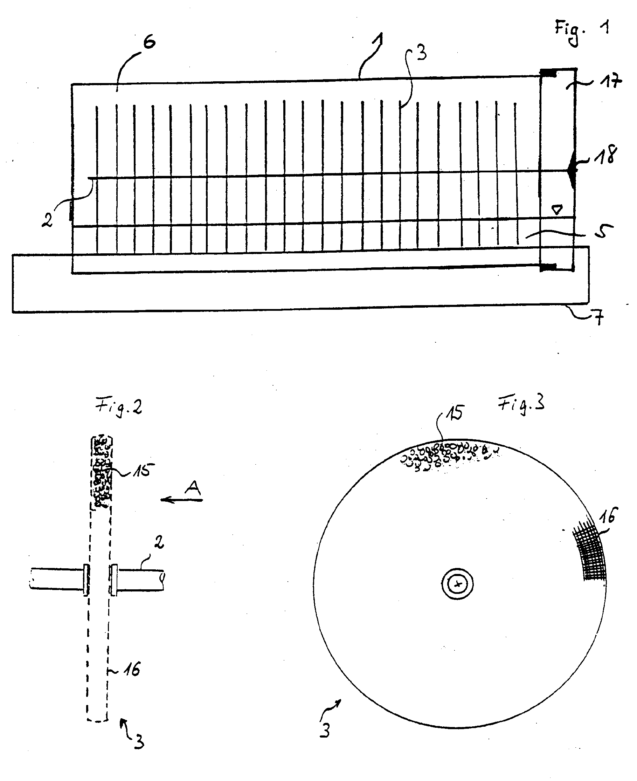

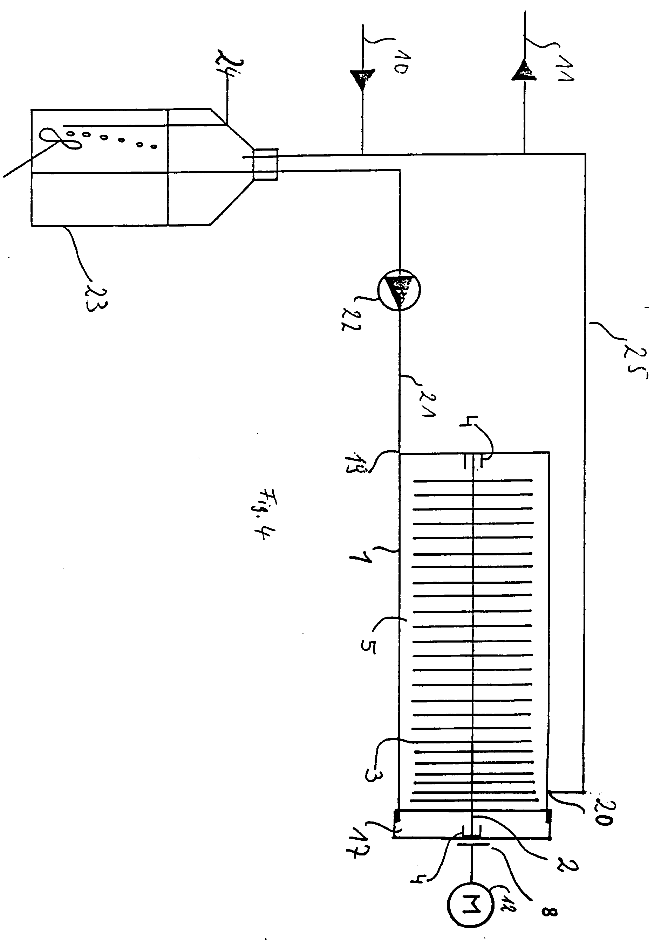

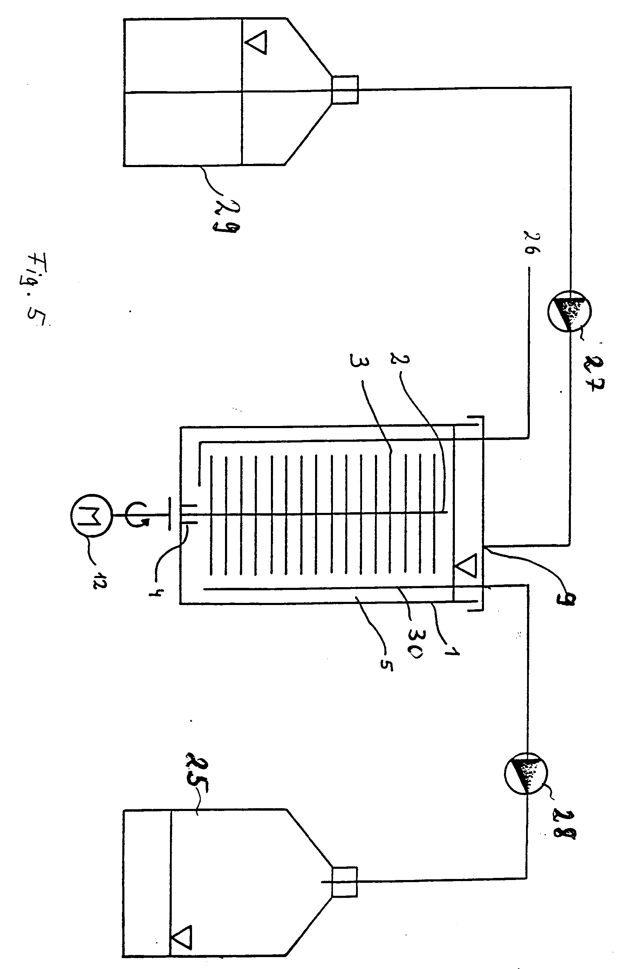

Die in den Figuren dargestellten Ausführungsbeispiele eines erfindungsgemäßen Reaktors besitzen jeweils ein zylindrisches Reaktionsgefäß 1, das mit horizontaler Gefäßachse (Figuren 1 und 4) oder vertikaler Gefäßachse (Fig. 5) angeordnet ist. In der Gefäßachse befindet sich eine Trägerwelle 2, die an den Stirnseiten des Reaktionsgefäßes 1 bei den Ausführungsformen der Figuren 4 und 5 drehbar gelagert ist. Auf der Trägerwelle 2 sind als Umhüllungen für einen partikelförmigen Füllstoff 15 mit bioaktiver Oberfläche Haltesiebe 16 befestigt, in welchen jeweils ein Perlpolymerisat als Füllstoff 15, das mit beispielsweise Wachstumsfaktoren überzogen ist, in Schichtform angeordnet ist. Durch die Haltesiebe 16 wird der Füllstoff 15 in paketförmigen Schichten 3 auf der Trägerwelle 2 gehalten. Die Haltesiebe 16 sind so geformt, daß die paketförmigen Schichten 3 des Perlpolymerisats 15 eine kreisrunde Form haben, die an den zylindrischen Innenraum des Reaktionsgefäßes 1 angepaßt ist. Die Paketschichten 3 des Perlpolymerisats 15 sind in Abständen voneinander auf der Trägerwelle 2 nebeneinander (Figuren 1 und 4) bzw. übereinander (Fig. 5) angeordnet. Die Trägerwelle 2 ist mit den daran gelagerten Haltesieben 16 und dem Perlpolymerisat gegenüber einem im Reaktionsgefäß 1 befindlichen Medium 5, z.B. einer Nährlösung, drehbar angeordnet. Die Abstände der Paketschichten 3 voneinander können innerhalb eines relativ breiten Bereiches in der Größenordnung von 0,1 bis 10 mm gewählt werden.

Zur Beschickung der Haltesiebe 16 mit dem Perlpolymerisat kann die Trägerwelle 2 zusammen mit den daran befestigten Haltesieben 16 dem Reaktionsgefäß 1 entnommen werden. Hierzu kann beispielsweise ein Behälteraufsatz 17, in welchem die Trägerwelle 5 gelagert ist, vom übrigen Teil des Reaktionsgefäßes 1 entfernt werden, so daß eine problemlose Beschickung der Haltesiebe 16 mit dem Perlpolymerisat ermöglicht ist.

Beim Ausführungsbeispiel 1 ist die Trägerwelle 2 drehfest mit dem Reaktionsgefäß 1 bzw. dem vom übrigen Gefäß abnehmbaren Behälteraufsatz 17 an einer Befestigungsstelle 18 verbunden. Bei den Ausführungsformen der Figuren 4 und 5 kann die Trägerwelle 2 aus ihren Wellenlagern 4 nach dem Abnehmen des Behälteraufsatzes 17 (Fig. 4) bzw. Öffnen des Reaktionsbehälters 1 gelöst werden.

Die Beschickung der Haltesiebe 16 mit dem Füllstoff muß nicht steril vor sich gehen. Die Sterilisierung kann nach dem Einsetzen der mit dem Perlpolymerisat 15 beschickten Haltesiebe 16 in das Reaktionsgefäß 1 beim Autoklavieren durchgeführt werden. Anschließend erfolgt dann die Zuführung von Zellkulturmedium und das Zellwachstum auf dem Perlpolymerisat. Nach der Zellkultur kann durch entsprechende biologische Prozesse beispielsweise die Produktion von Impfstoffen, Enzymen, Hormonen, Antikörpern und anderen Proteinen unter Zuhilfenahme der gewonnenen Zellkulturen erfolgen. Es kann jedoch auch eine Behandlung von fließfähigem Medium, beispielsweise von Blut, insbesondere Blutreinigung, in dem Reaktionsgefäß 1 durchgeführt werden.

Bei dem in der Fig. 1 dargestellten Ausführungsbeispiel ist das zylindrische Reaktionsgefäß 1 nach Art einer Rollerflasche ausgebildet. Die in den Haltesieben 16 vorgesehenen Schichten des partikelförmigen Füllstoffes 15 sind vertikal nebeneinander längs der horizontal liegenden Trägerwelle 2 im Innern des Reaktionsgefäßes 1 angeordnet. Die Trägerwelle 2 ist an der Befestigungsstelle 18 drehfest mit dem Reaktionsgefäß bzw. mit dem Behälteraufsatz 17 verbunden. In das Reaktionsgefäß 1 ist das Medium 5, beispielsweise eine Nährlösung, eingefüllt. Durch Rotation des Reaktionsgefäßes 1 auf einer Rollerapparatur 7 um die Gefäßachse wird die Trägerwelle 2 zusammen mit den Schichten des partikelförmigen Füllstoffes 15 mitgedreht. Die auf der Trägerwelle 2 fixierten Schichten 3 mit dem partikelförmigem Füllstoff 15, der gegebenenfalls an seiner Oberfläche Wachstumsfaktoren aufweist, werden beim Drehen in die Nährlösung 5 eingetaucht. Der übrige Teil des Gefäßinnern ist ein Gasraum 6, in welchem, nachdem die Füllstoffschichten das Medium 5 wieder verlassen haben, ein Gasaustausch, beispielsweise mit O₂ oder CO₂ im Gasraum 6 stattfindet.

Nach Verbrauch des Nährmediums 5 bzw. nach Beendigung der Reaktion, kann dann das Medium 5 ausgetauscht werden.

Bei dem in der Fig 4 dagestellten Ausführungsbeispiel wird ebenfalls ein zylindrisches Reaktionsgefäß 1 verwendet. Dieses Reaktionsgefäß 1 ist jedoch ortsfest angeordnet. Die Trägerwelle 2 mit den daran fixierten Schichten des Perlpolymerisats bzw. Füllstoffs 15 sind gegenüber dem Reaktionsgefäß 1 drehbar angeordnet. Hierzu kann ein Antriebsmotor 12 für den Drehantrieb der Trägerwelle 2 vorgesehen sein. Die Trägerwelle 2 ist hierzu drehbar in Wellenlagern 4 an dem Reaktionsgefäß 1 gelagert. Der Drehantrieb kann über eine Kupplung 8 erfolgen.

Das Reaktionsgefäß 1 ist mit einem Anschluß 19 für eine Mediumzufuhr und einem Anschluß 20 für eine Mediumabfuhr ausgestattet. Der Anschluß 19 für eine Mediumzufuhr, insbesondere eine Nährmediumzufuhr, ist über eine Leitung 21, in welcher eine Pumpe 22 vorgesehen ist, mit einem Konditionierungstank 23 verbunden. In diesen Konditionierungstank werden die Nährmediumszusammensetzung und verschiedene andere Parameter des Nährmediums, wie z.B. pH-Wert, pO₂ und dergleichen eingestellt. Eine Begasung des Konditionierungstanks 23 kann hierbei über eine Begasungsleitung 24 erfolgen. Zur kontinuierlichen Verfahrensführung wird mit Hilfe der Pumpe 22 und über die Leitung 21 und den Anschluß 19 ständig frisches Nährmedium in das Innere des Reaktionsgefäßes 1 zugeführt. Verbrauchtes Nährmedium wird über den Anschluß 20 und eine Leitung 25 abgeführt. Der Zufluß frischer Nährlösung erfolgt über eine Leitung 10 in den Konditionierungstank 23 und der Abfluß der verbrauchten Nährlösung erfolgt über eine Abflußleitung 11. Die Begasung zur Konditionierung der Nährstoffzusammensetzung im Konditionierungstank erfolgt über eine Begasungsleitung 24.

Anstelle der horizontalen Anordnung des Reaktionsgefäßes 1, welche in der Fig. 4 gezeigt ist, kann das Reaktionsgefäß 1 auch in vertikaler Stellung für den kontinuierlichen Betrieb eingesetzt werden.

Das Reaktionsgefäß 1 kann sowohl für die Zellkultur bzw. das Zellwachstum als auch für die Produktion von Zellprodukten der dabei gewonnenen Zellen verwendet werden. Ferner kann die in der Fig. 4 dargestellte Anordnung auch als "künstliches Organ", beispielsweise künstliche Leber, verwendet werden. Hierbei können schädliche Stoffe in biologischen Flüssigkeiten, beispielsweise im Blut, entgiftet oder umgewandelt werden. Die zu entgiftende Flüssigkeit wird dann in einem Behälter, welcher anstelle des Konditionierungstanks 23 in der Anordnung vorgesehen ist, bereitgehalten und über die Leitung 21 und den Anschluß 19 in den Reaktionsbehälter 1 eingebracht. Die entgiftete Flüssigkeit wird dann über den Anschluß 19 und die Leitung 25 sowie die Abflußleitung 11 in ein nicht näher dargestelltes Gefäß weitergeleitet.

Im Falle der Produktion von Zellprodukten werden die Zellprodukte ebenfalls über den Anschluß 20 und die Leitung 25 sowie die Abflußleitung 11 in ein nicht näher dargestelltes Erntegefäß eingebracht.

Bei dem in der Fig. 5 dargestellten Ausführungsbeispiel erfolgt die Konditionierung in dem Reaktionsgefäß 1. Es ist dann kein Konditionierungstank mehr erforderlich. Frische Nährlösung wird hierbei in einem Vorratsgefäß 29 in Bereitschaft gehalten und mittels einer Pumpe 27 über einen Nährlösungszufluß 9 dem Reaktionsgefäß 1 zugeführt. Die Konditionierung der Nährstoffzusammensetzung und der Einstellung von pH-Wert, pO₂ usw. erfolgt im Reaktionsgefäß 1. Zur Begasung, insbesondere zur Zufuhr von O₂ und CO₂ , dient eine Begasungsleitung 26. Über einen Abfluß 30 erfolgt die Entnahme verbrauchter Nährlösung. Mit Hilfe einer Pumpe 28 kann diese in einen Vorratsbehälter 25 gebracht werden. Bei der Produktion von Zellprodukten dient der Behälter 25 als sogenanntes Erntegefäß, in welchem die über den Abfluß 30 dem Reaktionsgefäß 1 entnommenen Zellprodukte von der Pumpe 28 dem Gefäß 25 zugeführt werden. Im Falle der Verwendung der in Fig. 5 gezeigten Anordnung als künstliches Organ wird die zu entgiftende Flüssigkeit aus dem Vorratsbehälter 29 zur Behandlung in das Reaktionsgefäß 1 eingebracht, und die entgiftete Flüssigkeit wird dann aus dem Reaktionsgefäß 1 über den Abfluß 30 in den Behälter 25 durch die Pumpe 28 befördert.

Die in den Figuren 4 und 5 gezeigten Anordnungen können in sämtlichen Verfahrensweisen, die in der Biotechnologie möglich sind, betrieben werden, d.h. im sogenannten Batch-Betrieb, im Feed-Batch-Betrieb, sowie im kontinuierlichen Betrieb.

## Patentansprüche

1. Reaktor zur Durchführung von biologischen in vitro-Prozessen mit einem zylindrischen Reaktionsgefäß, in welchem ein partikelförmiger Füllstoff, dessen Oberfläche bioaktiv ist, in Schichten senkrecht zur Gefäßachse angeordnet ist, sowie einem Einlaß und einem Auslaß für ein fließfähiges Medium, das an den Schichten des Füllstoffs vorbeiströmt, dadurch gekennzeichnet, daß
- der partikelförmige Füllstoff (15) in jeder der Schichten (3) durch eine Umhüllung (16) umfaßt ist, welche für eine Diffusion des Nährmediums (5) durchlässig ist;
- die Schichten des Füllstoffs (15) an einer Trägerwelle (2), die in der Behälterachse angeordnet ist, fixiert sind; und
- die Schichten (3) des Füllstoffs (15) gegenüber dem im Reaktionsgefäß (1) vorhandenen Medium (5) drehbar gelagert sind.

2. Reaktor nach Anspruch 1, dadurch gekennzeichnet, daß die Umhüllung (16) für die Diffusion von Zellen durchlässig ist.

3. Reaktor nach Anspruch 1 oder 2, dadurch gekennzeichnet, daß die Trägerwelle (2) am Reaktionsgefäß (1) drehbar gelagert ist.

4. Reaktor nach Anspruch 1 oder 2, dadurch gekennzeichnet, daß die Trägerwelle (2) am drehbaren Reaktionsgefäß (1) mit dem Reaktionsgefäß mitdrehend befestigt ist.

5. Reaktor nach einem der Ansprüche 1 bis 4, dadurch gekennzeichnet, daß die Umhüllung (16) als Haltesieb mit einer Maschenweite von 40 Mikron (µm) bis 100 Mikron (µm) ausgebildet ist.

6. Reaktor nach einem der Ansprüche 1 bis 5, dadurch gekennzeichnet, daß der partikelförmige Füllstoff (15) ein Perlpolymerisat mit einer Perlgröße >40 Mikron (µm) ist.

7. Reaktor nach einem der Ansprüche 1 bis 6, dadurch gekennzeichnet, daß die Schichten (3) des Füllstoffs (15) in axialer Richtung des Reaktionsgefäßes (1) jeweils eine Dicke von 1000 Mikron (µm) bis 2000 Mikron (µm) aufweisen.

8. Verfahren zur Durchführung eines biologischen in vitro-Prozesses, bei dem ein Reaktor nach einem der Ansprüche 1 bis 7 verwendet wird, dadurch gekennzeichnet, daß das Reaktionsgefäß (1) mit den darin angeordneten Schichten aus partikelförmigem Füllstoff mit bioaktiver Oberfläche, insbesondere Wachstumsfaktoren an der Oberfläche für eine Zellkultur verwendet wird.

9. Verfahren nach Anspruch 8, dadurch gekennzeichnet, daß nach dem Einsetzen der Schichten aus dem partikelförmigen Füllstoff mit bioaktiver Oberfläche in das Reaktionsgefäß das Gefäßinnere autoklaviert wird und dann mit Zellkulturmedium gefüllt und das Zellwachstum durchgeführt wird.

10. Verfahren nach Anspruch 8 oder 9, dadurch gekennzeichnet, daß in dem Reaktionsgefäß, in welchem das Zellwachstum erfolgt, die Produktion von Zellprodukten, insbesondere Stoffwechselprodukten der Zellen, durchgeführt wird.

11. Verfahren nach einem der Ansprüche 8 bis 10, dadurch gekennzeichnet, daß in dem Reaktionsgefäß, in welchem das Zellwachstum erfolgt ist, fließfähiges zu behandelndes Medium an den mit den Zellen bewachsenen Schichten des Füllstoffs vorbeigeleitet wird.

12. Verfahren nach einem der Ansprüche 8 bis 11, dadurch gekennzeichnet, daß der Füllstoff mit Säugetierzellen bewachsen wird.

13. Verfahren nach einem der Ansprüche 8 bis 12, dadurch gekennezichnet, daß die Konditionierung des Zellkulturmediums außerhalb des Reaktionsgefäßes erfolgt und das konditionierte Zellkulturmedium in das Reaktionsgefäß eingeleitet wird.

14. Verfahren nach einem der Ansprüche 8 bis 12, dadurch gekennzeichnet, daß die Konditionierung des Zellkulturmediums innerhalb des Reaktionsgefäßes durchgeführt wird.

15. Verfahren nach einem der Ansprüche 8 bis 14, dadurch gekennzeichnet, daß der partikelförmige Füllstoff mit Hepatozyten bewachsen wird, und durch das Reaktionsgefäß zu reinigendes Blut geleitet wird.

16. Verwendung eines Reaktors nach einem der Ansprüche 1 bis 7 und eines Verfahrens nach einem der Ansprüche 8 bis 15 als künstliche Leber.

## Claims

1. A reactor for carrying out biological in vitro processes comprising a cylindrical reaction vessel in which a filler in particle form, whose surface is bioactive, is arranged in layers perpendicularly to the axis of the vessel, and an inlet and an outlet for a medium which is capable of flow and which flows past the layers of the filler, characterised in that
- the filler (15) in particle form is enclosed in each of the layers (3) by a covering (16) which is permeable for diffusion of the nutrient medium (5);
- the layers of the filler (15) are fixed to a carrier shaft (2) which is arranged on the axis of the container; and
- the layers (3) of the filler (15) are mounted rotatably relative to the medium (5) present in the reaction vessel (1).

2. A reactor according to claim 1 characterised in that the covering (16) is permeable for the diffusion of cells.

3. A reactor according to claim 1 or claim 2 characterised in that the carrier shaft (2) is mounted rotatably on the reaction vessel (1).

4. A reactor according to claim 1 or claim 2 characterised in that the carrier shaft (2) is fixed to the rotatable reaction vessel (1) to rotate therewith.

5. A reactor according to one of claims 1 to 4 characterised in that the covering (16) is in the form of a holding mesh with a mesh size of from 40 microns (µm) to 100 microns (µm).

6. A reactor according to one of claims 1 to 5 characterised in that the filler (15) in particle form is a bead polymer with a bead size >40 microns (µm).

7. A reactor according to one of claims 1 to 6 characterised in that the layers (3) of the filler (15), in the axial direction of the reaction vessel (1), are each of a thickness of from 1000 microns (µm) to 2000 microns (µm).

8. A method of carrying out a biological in vitro process in which a reactor according to one of claims 1 to 7 is used characterised by using the reactor vessel (1) with the layers arranged therein of filler in particle form with a bioactive surface, in particular growth factors at the surface, for a cell culture.

9. A method according to claim 8 characterised in that after insertion of the layers of the filler in particle form with a bioactive surface into the reaction vessel, the vessel interior is autoclaved and then filled with cell culture medium and cell growth is effected.

10. A method according to claim 8 or claim 9 characterised in that the production of cell products, in particular metabolisn products of the cells, is effected in the reaction vessel in which cell growth is effected.

11. A method according to one of claims 8 to 10 characterised in that in the reaction vessel in which cell growth is effected, medium to be treated, which is capable of flow, is guided past the layers of the filler with the cell growth thereon.

12. A method according to one of claims 8 to 11 characterised in that the filler has mammal cell growth thereon.

13. A method according to one of claims 8 to 12 characterised in that conditioning of the cell culture medium is effected outside the reaction vessel and the conditioned cell culture medium is introduced into the reaction vessel.

14. A method according to one of claims 8 to 12 characterised in that conditioning of the cell culture medium is performed within the reaction vessel.

15. A method according to one of claims 8 to 14 characterised in that the filler in particle form has hepatocyte growth thereon and blood to be cleaned is passed through the reaction vessel.

16. Use of a reactor according to one of claims 1 to 7 and a method according to one of claims 8 to 15 as an artificial liver.

## Revendications

1. Réacteur pour la réalisation de processus biologiques in vitro, avec un réacteur cylindrique, dans lequel une charge en forme de particules, dont la surface est bioactive, est disposée en couches à la perpendiculaire de l'axe du réacteur, et avec une admission et une évacuation pour un milieu fluide, qui balaye les couches de la charge, caractérisé en ce que
- la charge (15) en forme de particules est enrobée dans chacune des couches (3) par une enveloppe (16), perméable pour une diffusion du milieu nutritif (5),
- les couches de la charge (15) sont fixées sur un arbre porteur (2), disposé dans l'axe du réacteur, et en ce que
- les couches (3) de la charge (15) sont logées avec une possibilité de rotation par rapport au milieu (5), présent dans le réacteur (1).

2. Réacteur suivant la revendication 1, caractérisé en ce que l'enveloppe (16) est perméable pour la diffusion de cellules.

3. Réacteur suivant l'une des revendications 1 et 2, caractérisé en ce que l'arbre porteur (2) est monté avec une possibilité de rotation sur le réacteur (1).

4. Réacteur suivant l'une des revendications 1 et 2, caractérisé en ce que l'arbre porteur (2) est fixé sur le réacteur (1) rotatif, avec lequel il peut tourner.

5. Réacteur suivant l'une quelconque des revendications 1 à 4, caractérisé en ce que l'enveloppe (16) est réalisée sous forme de crible de retenue, d'une ouverture de maille de 40 à 100 microns (µm).

6. Réacteur suivant l'une quelconque des revendications 1 à 5, caractérisé en ce que la charge (15) en forme de particules est un polymérisat en perles, d'une grosseur de perles de plus de 40 microns (µm).

7. Réacteur suivant l'une quelconque des revendications 1 à 6, caractérisé en ce que les couches (3) de la charge (15) présentent respectivement une épaisseur de 1000 à 2000 microns (µm), dans le sens axial du réacteur (1).

8. Procédé pour la réalisation d'un processus biologique in vitro, dans lequel est utilisé un réacteur suivant l'une quelconque des revendications 1 à 7, caractérisé en ce que le réacteur (1), avec les couches qu'il contient de charge en forme de particules, dotée d'une surface bioactive, de facteurs de croissance notamment en surface, est utilisé pour une culture cellulaire.

9. Procédé suivant la revendication 8, caractérisé en ce que, après la mise en place des couches de la charge en forme de particules, dotée d'une surface bioactive, dans le réacteur, l'intérieur de ce dernier est passé à l'autoclave, puis rempli de milieu de culture cellulaire, et le développement des cellules est assuré.

10. Procédé suivant l'une des revendications 8 et 9, caractérisé en ce que la production de produits cellulaires, de produits du métabolisme des cellules notamment, est assurée dans le réacteur, dans lequel se déroule le développement de cellules.

11. Procédé suivant l'une quelconque des revendications 8 à 10, caractérisé en ce qu'un milieu fluide à traiter est envoyé sur les couches de la charge, couvertes des cellules, dans le réacteur dans lequel se déroule le développement cellulaire.

12. Procédé suivant l'une quelconque des revendications 8 à 11, caractérisé en ce que la charge est couverte de cellules de mammifères.

13. Procédé suivant l'une quelconque des revendications 8 à 12, caractérisé en ce que le conditionnement du milieu de culture cellulaire est assuré à l'extérieur du réacteur, le milieu de culture conditionné étant envoyé dans le réacteur.

14. Procédé suivant l'une quelconque des revendications 8 à 12, caractérisé en ce que le conditionnement du milieu de culture cellulaire est assuré à l'intérieur du réacteur.

15. Procédé suivant l'une quelconque des revendications 8 à 14, caractérisé en ce que la charge en forme de particules est couverte d'hépatocytes, du sang à épurer étant envoyé au travers du réacteur.

16. Application d'un réacteur, suivant l'une quelconque des revendications 1 à 7, et d'un procédé, suivant l'une quelconque des revendications 8 à 15, sous forme de foie artificiel.
